# EUROPEAN PATENT SPECIFICATION

(11) **EP 0 795 307 B1**
(45) Date of publication and mention of the grant of the patent: **14.04.2004**
(21) Application number: 97301317.0
(22) Date of filing: 27.02.1997
(51) Int. Cl.: A61F 13/62

(54) **Disposable nappies with hook and loop fastener and method of manufacturing the nappy**
Wegwerfwindel mit Haftverschluss und Herstellungsverfahren einer Wegwerfwindel
Couche à jeter avec fermeture à crochets et boucles et methode de fabrication d'un couche à jeter

(30) Priority: 27.02.1996 GB 9604062; 29.05.1996 GB 9611140
(43) Date of publication of application: 17.09.1997
(73) Proprietor: YKK Europe Limited, London EC1V 8AN (GB)
(72) Inventor: Kato, Hisanori, 60488 Frankfurt (DE)
(74) Representative: Luckhurst, Anthony Henry William

(56) References cited:
- EP-A- 0 319 249
- EP-A- 0 324 578
- WO-A-93/19713
- WO-A-95/30397
- US-A- 5 019 073

## Description

The present invention relates to a disposable nappy having a hook and loop a fastener and a method of manufacturing the nappy.

Disposable nappies using fastening tapes which are secured by pressure sensitive adhesive are well known. These suffer from the drawback that contamination with powder, oil, etc. when fitting the nappy impairs the fastening. Also, it is difficult to provide a re-usable fastening tape which allows the nappy to be checked and re-fitted.

Disposable nappies using hook and loop fastening systems have been proposed, for example in US 4 894 060. A difficulty with the hook and loop fastening system is the need to keep the cost of the fastener very low, and to allow automated manufacture of the fasteners and to attach the fasteners automatically to the nappy.

Other Examples of hook and loop fastening systems for disposable nappies have been disclosed in EP-A-0324578 and EP-A-0319249 where the hook portion of the fastener is released for use by peeling it off from an adhesive layer. Also, in W095/30397 a hook portion is used to fasten the nappy in use by hooking on to the nappy fabric.

The invention provides a disponible nappy as set forth is claim 1, and a method of manufacturing the nappy or set forth in claim 3.

Other, preferred, features of the invention will be apparent from the following description and the accompanying claims.

The invention will be further described by way of example, with reference to the accompanying drawings, in which:
Figure 1 shows a nappy having fasteners in accordance with a first embodiment of the invention;
Figures 2 and 3 show a detail of the fastener of Figure 1;
Figures 4 to 8 show steps during the manufacture of fasteners of the first embodiment, in accordance with the invention, and mounting them on a nappy;
Figure 9 shows a second embodiment of a hook portion of a hook and loop fastener in accordance with the invention; and
Figures 10 to 12 show steps in the manufacture of the hook portion of the second embodiment.
Figures 13 and 14 show yet another embodiment of a hook portion of a hook and loop fastener in accordance with the invention; and
Figure 15 shows a continuous strip from which the hook portion of Figure 13 can be cut.
Figure 16 shows the strip of Figure 15 rolled along its length.

The drawings, in particular the thickness of the layers, are not drawn to scale, in order to better illustrate the adhesive layers.

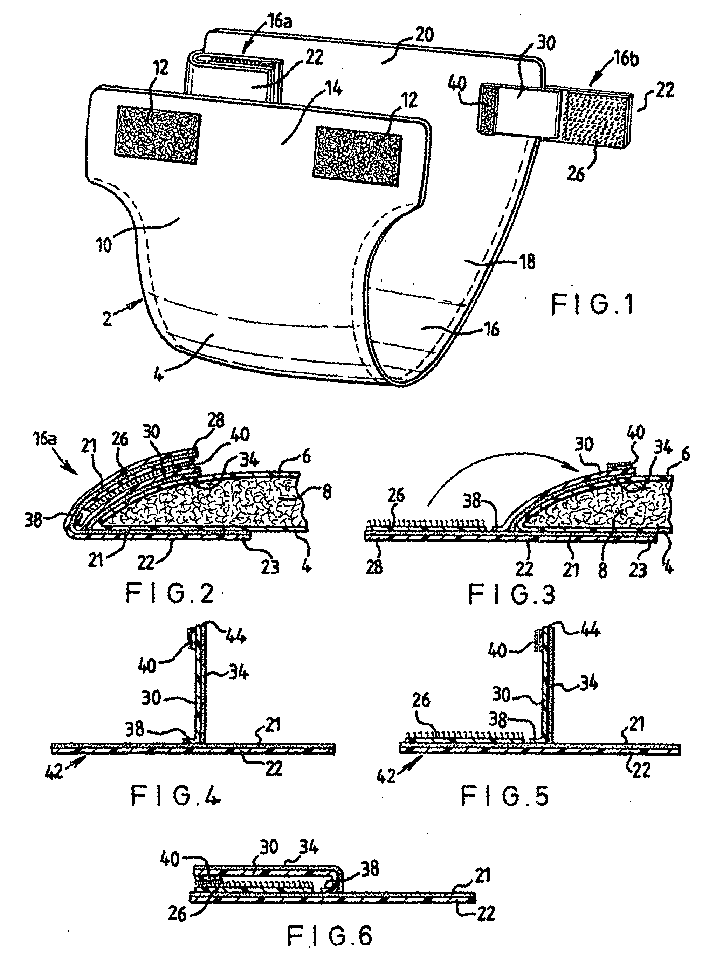

Figure 1 shows a disposable nappy 2 which has a laminated structure comprising a generally impermeable outside layer 4, a porous inside layer 6 and a layer 8 of absorbent material sandwiched between. The general structure of such nappies is well known in the art and the invention described herein is not limited to any particular structure, but rather is concerned with a fastener for securing the nappy to a baby or infant.

A front section 10 of the nappy has two panels 12 of loop material adhered to the outer layer 4 at a waist region 14. Typically the loop material has a woven polypropylene base with loops woven into one surface. Many varieties of loop material for hook and loop fastener systems are known, and it is desired to use a loop material which is inexpensive and also soft to the touch.

Two hook assemblies 16a, 16b are secured to the rear section 18 of the nappy 2 at the waist region 20. One assembly 16a is shown in the closed position, in which the nappy is packed for the user, while the other assembly 16b has been opened ready for use.

Referring to Figures 2 and 3, hook fastener assembly 16 has a base tape 22 formed of flexible polymeric strip having a pressure sensitive adhesive layer 21 on one surface for securing the base tape 22 at one end 23 to the outer layer 4 of the nappy 2. A panel 26 of hook material is secured to the other end 28 of the strip 22 by the adhesive layer 21. A cover 30 of the same material as the base tape 22 has a pressure sensitive adhesive layer 34 on one surface and is secured at one end 38 to the base tape 22 between the body of the nappy and the hook panel 26 and is secured to the inside layer 6 of the nappy by the adhesive layer 34 A small panel 40 of loop material is attached, by adhesive, to the cover 30 and hooks on the panel 26 engage the loop material panel 40 to hold the fastener 16 in the closed position (Figure 2). To use the fastener, the panel 26 is peeled away from the loop material panel 40, to expose the hooks and make them ready for engagement with the loop material panels 12 (Figure 3).

The hook panel 26 is preferably formed of small moulded hooks having a rounded profile, to feel comfortable to the touch. Mushroom shaped hooks and cut filament type hooks could also be used

The manufacture of the hook fasteners 16a, 16b, will now be described with reference to Figures 4 to 8.

In Figure 4 a continuous base tape 22 has a pressure sensitive adhesive layer 21 on one surface. The other surface 42 is treated so that it will not normally stick to the adhesive layer 21. For example a silicone coating may be applied. Such adhesive tapes are well known in the art.

A cover panel 30 is formed from the same polymeric material as base tape 22 and has a layer 34 of pressure sensitive adhesive on one surface and is attached at one end 38 to the base tape 22 by pressing onto the adhesive layer 21. A small panel 40 of loop material is adhered to the outer end 44 of the cover sheet. The loop material can be manufactured with a coating of pressure sensitive adhesive on its underside, to adhere to the panel 30.

It will be appreciated that the cover sheet 30 may be formed by applying the loop panels 40 at predetermined positions along a length of the cover sheet material and then cutting the strip material to form the discrete panels 30.

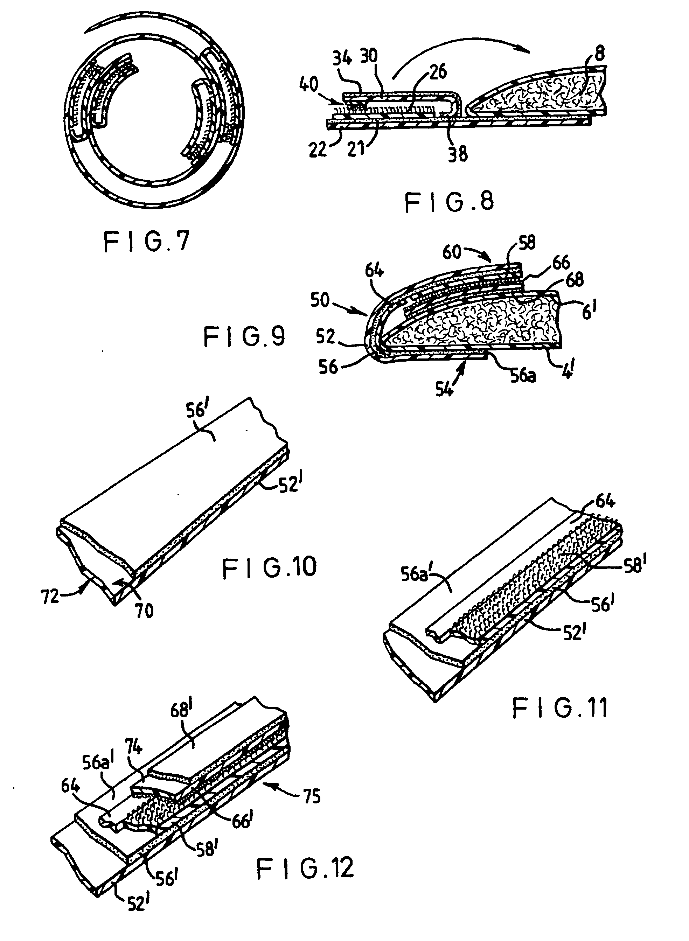

Referring to Figure 5, a hook panel 26 is secured to the base tape 22 by pressing onto the adhesive layer 21, adjacent the cover sheet 30. The cover sheet 30 is then folded over to engage the loop part 40 with the hook panel 26 (Figure 7). The continuous base tape 22 can thus be formed with numerous fasteners 16 along its length and rolled for storage or shipment to the nappy manufacturer. The silicone treated surface 42 of the base tape lays against or faces the adhesive layer 34 on the cover sheet 30, and the adhesive layer 21, and so the tape can be unrolled without damaging the fasteners 16. At the nappy manufacturing stage, the base tape 22 is cut into discrete fastener lengths and secured to the outer layer 4 of the nappy 2 (Figure 8). The fastener 16 is then folded over, to press the adhesive layer 34 of the cover sheet 30 against the inner layer 6 of the nappy as shown in Figure 2. As explained above, the user peels the hook part 26 away from the loop panel 40 to make the fastener ready for use.

Various modifications may be made to the described embodiment. For example, the hook panel 26 may be integrally formed with the base material 21 by forming areas of hooks at staggered intervals along a continuous length of base material, and applying adhesive to the regions between the areas of hooks. The cover 30 need not be attached at its end 38 to the base tape 22.

The loop panel 40 may be provided inward of the end of the cover sheet 30, leaving a flap at the outer end of the hook material panel 26, to facilitate peeling of the panel 26 away from the loop panel 40.

Also, the panels 12 may be of hook material in which case panels 26 are of loop material, and panels 40 of hook material.

Figure 9 shows a second embodiment of a hook portion of a hook and loop fastener, in the closed position. The hook portion 50 of Figure 9 comprises a flexible polymeric base tape 52 which is secured at one end 54 by a portion 56a of a layer of pressure sensitive adhesive 56 to the outside layer 4' of a nappy. A panel 58 of hook material of a hook and loop fastener is secured to the other end 60 of the base tape 52 by the layer of adhesive 56, and a spacing layer 64 of paper is positioned over the central portion of the adhesive 56 on the base tape 52. A loop portion 66 is secured to the inner layer 6' of the nappy by a pressure sensitive adhesive layer 68 and holds the panel 58, when the hook fastener portion 50 is in the closed position (c.f. the hook portion 16a of Figures 1 and 2).

The manufacture of the hook fastener portions 50 will be described with reference to Figures 10 to 12. In Figure 10 a continuous sheet 52' of base tape material has a coating of pressure sensitive adhesive 56' on one major surface 70, and the other surface 72 is coated with a silicone layer which will not normally adhere to the pressure sensitive adhesive 56'.

In Figure 11, a strip 58' of hook fastener panel material and a strip 64' of paper are adhered to the base tape material 52' by the pressure sensitive adhesive layer 56'.

In Figure 12, a strip 66' of loop material having a layer 68' of pressure sensitive adhesive on its back surface 74 is then secured over the strip 58' of hook fastener panel material.

The completed continuous strip 75 of Figure 12 can be rolled up in the length direction, because the silicone treated surface 72 of the backing material sheet 52' will contact the adhesive layer 68' on the loop material strip 66', and the exposed adhesive portion 56a' on the sheet 52'.

Alternatively, a cover sheet (not shown) treated with release agent, may be laid over the adhesive layer 68' and exposed region 56a' before rolling.

The manufactured roll can be shipped to the nappy manufacturer in this form and is unrolled and cut across its width for forming the fastener portions 50. The exposed adhesive portion 56a' is the pressed against the outer layer panel 4' of the nappy to secure the hook fastener portion in place and the hook fastener portion folded around the edge of the nappy to secure the loop panel 66 to the inner layer 6'.

Again, it will be appreciated that the loop panel 66 may be of smaller width than and/or stop short of the outer end 76 of the hook panel 58 to facilitate peeling of the panel 58 from the loop panel 66. Also, the panels 58 may be of loop material, and the panels 66 of hook material, or mixed hooks and loops may be provided on the panels.

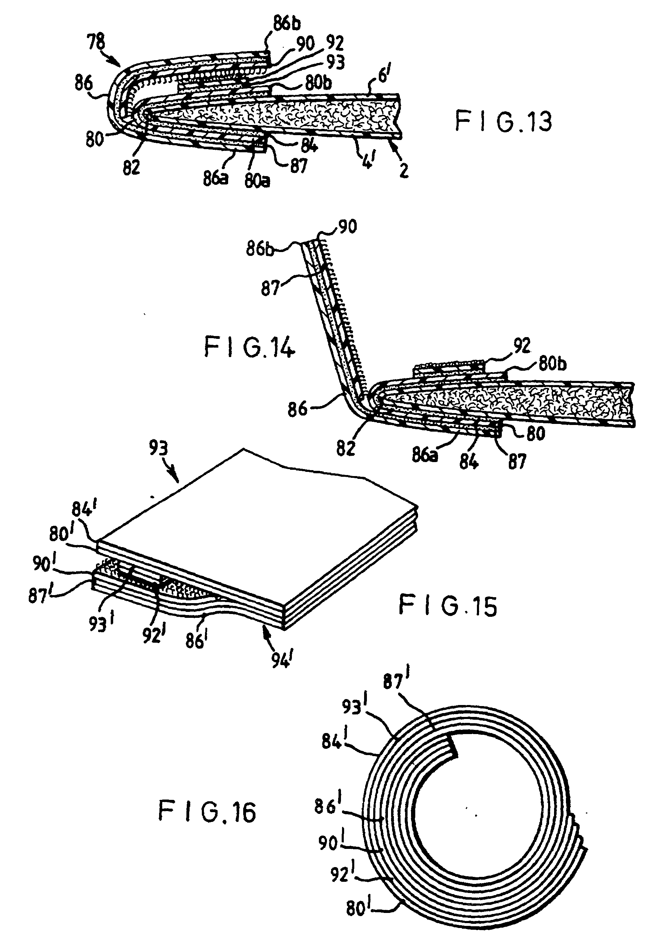

Referring to Figure 13, this embodiment is similar to the embodiment of Figure 9, but with the omission of the paper strip 64, and the addition of a relatively stiff backing strip 80 which adheres to the nappy.

The backing strip 80 extends around the edge 82 of the nappy 2 at the join between the layers 4', 6' and is adhered to the nappy by a pressure sensitive adhesive layer 84. A base tape 86 is adhered at one end 86a to an end 80a of the backing strip 80 by pressure sensitive adhesive 87 and carries a hook panel 90 which extends from the other, outer end 86b of the base tape 86 up to the edge 82 of the nappy. A loop panel 92 is attached by an adhesive layer 93 to the backing strip 80 a little short of the other end 80b of the backing strip 80, and the hook panel 90 and is releasably engaged with the loop panel 92.

Figure 14 shows the fastener 78 of Figure 13 opened out.

Figure 15 shows a continuous strip 93 which is cut across its width to form the discrete fasteners 78. The base tape 82' has its exposed surface 94' silicone treated so that the continuous strip can be rolled up for shipment. The continuous strip is then cut across its width to form the discrete fasteners.

Figure 16 shows the strip 93 rolled along its length. Again, it is emphasised that the thickness of the adhesive layers 84', 87', 93' is exaggerated in Figures 13 to 15 of the drawings, and has been reduced in Figure 16.

## Claims

1. A disposable nappy having a hook and loop fastener for fastening the nappy on a wearer, the fastener comprising:
a first strip of material (22, 52, 86) having a hook means (26, 58, 90) adhered to one surface of the strip (22, 52, 86) at one end thereof, the other end of the surface (22, 52, 86) of the first strip (22, 52, 86) being adhered to an outer surface (4, 4') of the rear section (18) of the nappy (2) at an edge of the waist region (20),
a second strip of material (30, 80) attached to the inner surface (6, 6') of the rear section (18) of the nappy at the waist region (20), and a first loop means (40, 66, 92) adhered to the outer facing surface of the second strip of material (30, 80),
the first strip (22, 52, 86) being folded between the said ends of the strip and the hook means (26, 58, 90) being engaged with the first loop means (40, 66, 92) prior to use of the nappy, and
a second loop means (12) provided on an outer surface (4) of a front section (10) of the nappy (2),
wherein, in use, the hook means (26, 58, 90) is disengaged from the first loop means (40, 66, 92) when the nappy is to be placed on a wearer, and the hook means (26, 58, 90) is engaged with the second loop means (12) to secure the nappy on the wearer.

2. A nappy as claimed in claim 1, wherein the hook part comprises moulded hooks.

3. A method of manufacturing the nappy as claimed in claim 1, the method including the use of a source of hook and loop parts, said source comprising a continuous elongate strip (22, 52, 86) of backing material having pressure sensitive adhesive (21, 56, 87) on one surface thereof, a continuous strip of the hook means (26, 58, 90) attached to the backing material (22, 52, 86) via the pressure sensitive adhesive (21, 56, 87) and of smaller width than the backing material (22, 52, 86), and a continuous strip of the first loop means (40, 66, 92) laying on and engaging the hook means (26, 58, 90), and a pressure sensitive adhesive (34, 68, 93) on the outer surface of the first loop means (40, 66, 92), the source being cut across its width to provide said hook and loop parts.

4. A method as claimed in claim 3, **characterised in that** the other surface of the backing material (22, 52, 86) is coated with a release agent, which will not permanently adhere to the pressure sensitive adhesive.

5. A method as claimed in claims 3 or 4, **characterised in that** a strip (80) of relatively stiff material overlays and is adhered to the fisrt loop means (92) and the exposed backing material (86) adjacent the first loop means (92), and is coated on its outer surface with a pressure sensitive adhesive (84).

## Patentansprüche

1. Wegwerfwindel, die einen Haftverschluss zum Festmachen der Windel an einem Träger aufweist, wobei der Haftverschluss Folgendes umfasst:
einen ersten Materialstreifen (22, 52, 86), der ein Hakenmittel (26, 58, 90) aufweist, das an einem Ende des Streifens an eine Oberfläche des Streifens (22, 52, 86) geklebt ist, wobei das andere Ende der Oberfläche (22, 52, 86) des ersten Streifens (22, 52, 86) an einem Rand des Taillenbereichs (20) an eine äußere Oberfläche (4, 4') des hinteren Abschnitts (18) der Windel geklebt ist,
einen zweiten Materialstreifen (30, 80), der am Taillenbereich (20) an der inneren Oberfläche (6, 6') des hinteren Abschnitts (18) der Windel befestigt ist, und ein erstes Ösenmittel (40, 66, 92), das an die äußere gegenüberliegende Oberfläche des zweiten Materialstreifens (30, 80) geklebt ist, wobei
der erste Steifen (22, 52, 86), der zwischen den Enden des Streifens und des Hakenmittels (26, 58, 90) gefaltet ist, vor der Verwendung der Windel mit dem ersten Ösenmittel (40, 66, 92) in Eingriff steht, und
ein zweites Ösenmittel (12), das an einer äußeren Oberfläche (4) eines vorderen Abschnitts (10) der Windel (2) bereitgestellt ist,
wobei das Hakenmittel (26, 58, 90) bei der Verwendung vom ersten Ösenmittel (40, 66, 92) gelöst wird, wenn die Windel einem Träger angelegt werden soll, und das Hakenmittel (26, 58, 90) mit dem zweiten Ösenmittel (12) in Eingriff gebracht wird, um die Windel am Träger zu befestigen.

2. Windel nach Anspruch 1, wobei der Hakenteil formgegossene Haken umfasst.

3. Verfahren zur Herstellung der Windel nach Anspruch 1,
wobei das Verfahren die Verwendung einer Quelle von Haken- und Ösenteilen beinhaltet, und diese Quelle Folgendes umfasst: einen fortlaufenden länglichen Streifen (22, 52, 86) eines Unterlagsmaterials, der an einer Oberfläche davon einen Haftklebstoff (21, 56, 87) aufweist, einen fortlaufenden Streifen des Hakenmittels (21, 56, 87), der über den Haftklebstoff (21, 56, 87) am Unterlagsmaterial (22, 52, 86) befestigt ist und eine geringere Breite als das Unterlagsmaterial (22, 52,86) aufweist, und einen fortlaufenden Streifen des ersten Ösenmittels (40, 66,92), der auf dem Hakenmittel (26, 58, 90) angeordnet ist und mit diesem in Eingriff steht, und einen Haftklebstoff (34, 68, 93) an der äußeren Oberfläche des ersten Ösenmittels (40, 66, 92), wobei die Quelle entlang ihrer Breite geschnitten wird, um die Haken- und Ösenteile bereitzustellen.

4. Verfahren nach Anspruch 3, **dadurch gekennzeichnet, daß** die andere Oberfläche des Unterlagsmaterials (22, 52, 86) mit einem Trennmittel beschichtet ist, das nicht dauerhaft am Haftklebstoff kleben wird.

5. Verfahren nach Anspruch 3 oder 4, **dadurch gekennzeichnet, daß** ein Streifen (80) eines verhältnismäßig steifen Materials das erste Ösenmittel (92) und das freiliegende Unterlagsmaterial (86) neben dem ersten Ösenmittel (92) bedeckt und daran geklebt ist und an seiner äußeren Oberfläche mit einem Haftklebstoff (84) beschichtet ist.

## Revendications

1. Couche jetable avec fermeture à crochets et à boucles pour attacher la couche sur un porteur, la fermeture comprenant :
une première bande de matière (22, 52, 86) ayant un moyen à crochets (26, 58, 90) collé sur une surface de la bande (22, 52, 86) à une extrémité de celle-ci, l'autre extrémité de la surface (22, 52, 86) de la première bande (22, 52, 86) étant collée sur une surface extérieure (4, 4') de la portion arrière (18) de la couche (2) sur un bord de la région de la taille (20),
une deuxième bande de matière (30, 80) fixée sur la surface intérieure (6, 6') de la portion arrière (18) de la couche dans la région de la taille (20), et un premier moyen à boucles (40, 66, 92) collé sur la surface orientée vers l'extérieur de la deuxième bande de matière (30, 80),
la première bande (22, 52, 86) étant pliée entre lesdites extrémités de la bande et le moyen à crochets (26, 58, 90) étant accroché au premier moyen à boucles (40, 66, 92) avant l'utilisation de la couche, et
un deuxième moyen à boucles (12) placé sur une surface extérieure (4) d'une portion avant (10) de la couche (2),
dans laquelle, en utilisation, le moyen à crochets (26, 58, 90) est décroché du premier moyen à boucles (40, 66, 92) quand la couche doit être placée sur un porteur, et le moyen à crochets (26, 58, 90) est accroché au deuxième moyen à boucles (12) pour attacher la couche sur le porteur.

2. Couche selon la revendication 1, dans laquelle la partie à crochets comprend des crochets moulés.

3. Procédé de fabrication d'une couche conforme à la revendication 1, le procédé comprenant l'utilisation d'une source de parties à crochets et à boucles, ladite source comprenant une bande allongée continue (22, 52, 86) de matière de support comportant un adhésif sensible à la pression (21, 56, 87) sur une de ses surfaces, une bande continue du moyen à crochets (26, 58, 90) fixée à la matière de support (22, 52, 86) au moyen de l'adhésif sensible à la pression (21, 56, 87) et de largeur inférieure à celle de la matière de support (22, 52, 86), et une bande continue du premier moyen à boucles (40, 66, 92) posée sur et accrochée au moyen à crochets (26, 58, 90), et un adhésif sensible à la pression (34, 68, 93) sur la surface extérieure du premier moyen à boucles (40, 66, 92), la source étant coupée dans le sens de la largeur pour donner lesdites parties à crochets et à boucles.

4. Procédé selon la revendication 3, **caractérisé en ce que** l'autre surface de la matière de support (22, 52, 86) est revêtue d'un agent anti-adhésif, qui n'adhère pas de façon permanente à l'adhésif sensible à la pression.

5. Procédé selon la revendication 3 ou 4, **caractérisé en ce qu'**une bande (80) de matière relativement rigide recouvre et est collée au premier moyen à boucles (92) et à la matière de support exposée (86) adjacente au premier moyen à boucles (92), et est revêtue sur sa surface extérieure d'un adhésif sensible à la pression (84).
